(19)

**Europäisches Patentamt**

**European Patent Office**

**Office européen des brevets**

(11)  **EP 4 647 741 B1**

(12)  **EUROPEAN PATENT SPECIFICATION**

(45) Date of publication and mention
of the grant of the patent:
**11.03.2026  Bulletin 2026/11**

(21) Application number: **24837281.5**

(22) Date of filing: **20.03.2024**

(51) International Patent Classification (IPC):
*G01N 21/359* (2014.01)   *G16C 20/20* (2019.01)
*G16C 60/00* (2019.01)   *G01N 5/04* (2006.01)
*G01N 25/20* (2006.01)   *G01N 21/31* (2006.01)

(52) Cooperative Patent Classification (CPC):
**G01N 33/02; G01N 21/31; G01N 21/3563;**
**G01N 21/359; G01N 25/20; G16C 20/20;**
G01N 2201/129

(86) International application number:
**PCT/CN2024/082571**

(87) International publication number:
**WO 2025/194360 (25.09.2025 Gazette 2025/39)**

(54)  **CIGARETTE TOBACCO BLEND COMPOSITION ANALYSIS METHOD BASED ON FUSION GRAPHS**

VERFAHREN ZUR ANALYSE DER ZUSAMMENSETZUNG VON ZIGARETTENTABAKMISCHUNGEN AUF BASIS VON FUSIONSGRAPHEN

PROCÉDÉ D'ANALYSE DE COMPOSITION DE MÉLANGE DE TABAC DE CIGARETTE SUR LA BASE DE GRAPHES DE FUSION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **19.03.2024   CN 202410311499**

(43) Date of publication of application:
**12.11.2025   Bulletin 2025/46**

(73) Proprietor: **CHINA TOBACCO YUNNAN INDUSTRIAL CO., LTD**
**Wuhua District**
**Kunming**
**Yunnan 650231 (CN)**

(72) Inventors:
• **YANG, Ji**
**Kunming, Yunnan 650231 (CN)**
• **YE, Ling**
**Kunming, Yunnan 650231 (CN)**
• **LI, Yong**
**Kunming, Yunnan 650231 (CN)**
• **WANG, Mingjing**
**Kunming, Yunnan 650231 (CN)**
• **WANG, Lei**
**Kunming, Yunnan 650231 (CN)**
• **ZHANG, Xingyue**
**Kunming, Yunnan 650231 (CN)**
• **JIANG, Wei**
**Kunming, Yunnan 650231 (CN)**
• **LI, Zhenjie**
**Kunming, Yunnan 650231 (CN)**
• **PENG, Qiyuan**
**Kunming, Yunnan 650231 (CN)**
• **XU, Yanqun**
**Kunming, Yunnan 650231 (CN)**
• **SU, Zhongbi**
**Kunming, Yunnan 650231 (CN)**

(74) Representative: **Gille Hrabal**
**Partnerschaftsgesellschaft mbB**
**Patentanwälte**
**Brucknerstraße 20**
**40593 Düsseldorf (DE)**

(56) References cited:
CN-A- 104 568 823    CN-A- 110 659 691
CN-A- 111 543 668    CN-A- 116 266 475
CN-A- 116 482 056    CN-A- 116 665 811

CN-A- 116 665 811 CN-A- 116 699 075
CN-B- 101 251 471 CN-B- 106 645 530

**Description**

TECHNICAL FIELD

The invention belongs to the technical field of tobacco, in particular to a method for analyzing the composition of coiled tobacco leaf groups based on fusion mapping.

BACKGROUND

The quality and style characteristics of cigarettes are mainly formed by product designers through the proportions of tobacco leaves of different origins, varieties and grades. It is usually necessary to rely on formula experience and sensory evaluation, and manually select 10-20 kinds of tobacco leaves from hundreds of stock grades of tobacco raw materials to design different formulas in different proportions. As a result, the composition of the coiled-tobacco leaf groups is extremely complicated, and it is difficult to analyze the composition of the unknown coiled-tobacco leaf groups manually. It is of great significance for the analysis of unknown tobacco and the design of leaf groups to analyze the composition of coiled tobacco leaves by means of instrument testing and using objective data and scientific technology.

In recent years, NIR technology has been widely used in tobacco quality analysis and evaluation, formulation design and maintenance because of its fast, efficient and abundant quality information. However, NIR analysis technology only considers the correlation between tobacco leaf quality and leaf composition under "static conditions," ignoring the quality characteristics under burning conditions during the consumption of cigarette products, and cannot truly represent the smoking quality of tobacco leaves, that is, the quality characteristics of smoke.

Thermogravimetric analysis (TG/DTA) can provide stable reaction conditions under programmed temperature conditions, and is the most ideal experimental tool for tobacco pyrolysis research. Derivative thermogravimetric methodology, also called the derivative thermogravimetric method, is derived from thermogravimetric analysis, and is a technique to record the first derivative of a TG curve with respect to temperature or time. The result of the experiment is a derivative thermogravimetric curve, that is, a DTG curve. The characteristics of DTG curves are: accurate reflection of the initial reaction temperature, maximum reaction rate temperature and reaction termination temperature of each weight loss stage; and the area of each peak on the DTG curve is proportional to the corresponding sample weight loss on the TG curve. When the TG curve is not obvious to some steps in the heating process, the DTG curve can be clearly distinguished. The main feature of thermogravimetric analysis is that it is highly quantitative and can accurately measure the mass change and the rate of change. It can be said that according to this feature, as long as the mass of a substance changes when it is heated, it can be studied by thermogravimetric analysis.

At present, the composition analysis of coiled tobacco leaves mostly adopts the combination of tobacco chemical composition analysis, flue gas chemical composition analysis, sensory evaluation and other means. The work intensity is high, the subjectivity is strong, and the conclusions drawn are vague and not referential.

CN116482056 discloses a method combining near-infrared characteristic spectrum data of tobacco leaves in a cigarette formula and the characteristic component data of the pyrolysis products using a multi-modal fusion algorithm. A convolutional neural network is used to obtain a tobacco leaf fusion feature matrix in the cigarette formula.

In order to solve the above problems, the invention is proposed.

SUMMARY

In order to solve the existing problems of composition analysis of coiled tobacco leaves, the invention constructs a fusion map that can fully reflect tobacco quality by fusing a near-infrared spectrum and a thermal analysis map. Furthermore, the difference correlation model of the fusion map was established to simulate and evaluate the coincidence between the analytic composition of the formula and the real leaf formula. In order to improve the generality of coiled-tobacco group analysis and the work efficiency of analysts, the invention uses the fusion spectrum to analyze and characterize the quality information of shredded cigarette tobacco. The invention includes a fusion graph differential correlation model and a formula analysis combination optimization algorithm to automatically search for the tobacco leaf ratio of the cigarette leaf group formula, uses objective data to analyze the composition of the cigarette leaf group. The composition and proportion of the leaf group formula can be clearly obtained, which is of great significance to the analysis and design of cigarette leaf group formulas.

The invention provides a method for analyzing a coiled tobacco leaf group composition based on fusion mapping. The specific steps are based on the ability of fusion maps to characterize tobacco quality and analyze the specific composition and formula ratio of competing cigarettes (cigarettes to be analyzed).

The technical scheme of the invention is as follows:

A method for analyzing the composition and proportion of tobacco leaf groups in a cigarette sample based on fusion mapping, characterized in that it includes the following steps: (1) preparation of a cigarette sample to be analyzed and a single-grade tobacco leaf sample; (2) construction of a fusion map for the cigarette sample to be analyzed and the single-grade tobacco leaf sample; and (3) analysis of the fusion map to obtain the composition and proportion of tobacco leaf in the cigarette sample to be analyzed.

Preferably, in Step (1), there is a single cigarette sample to be analyzed, with at least 50 single-grade tobacco leaf samples selected. Each sample is placed in a constant temperature and humidity environment of $(22 \pm 1)$ °C and $(60 \pm 2)$ % relative humidity for at least 48 hours for equilibrium. Generally, the cigarette sample is not less than 5 g, and the sample crushing mesh is not less than 100 mesh. Generally, no less than 50 typical single-grade tobacco samples are selected, and the information of the tobacco samples should cover different grades, different origins and different parts, and the smoking taste of typical single-grade tobacco samples is quite different. The tobacco sample shall not be less than 5 g, and the number of sample crushing mesh shall not be less than 100 mesh.

Preferably, in Step (2), the sub-steps of the fusion map construction of cigarette sample to be analyzed and single-grade tobacco samples are as follows:

Sub-step (21), Collecting near-infrared spectra: Weigh 3 g of the sample powder and place it in a sample cup, scan the spectrum in the 4000-9000 cm$^{-1}$ band, and repeat the operation 10 times for each sample to obtain a spectral average;

Sub-step (22), Collecting a thermal analysis map: the samples are respectively heated in thermogravimetric (TG) crucibles by a procedure including: an initial temperature of 50 °C, heating rate of 10 °C/min; final temperature at 900 °C, and constant temperature at 900 °C for 5 min; the protection gas and reaction gas are nitrogen, and the flow rate is 20 mL/min. Taking temperature (°C) as the X-axis and mass change (%) as the Y-axis, deriving the TG data, obtaining the first derivative of temperature, then obtaining derivative thermogravimetric (DTG) data of differential weight loss, and forming the thermal analysis map.

Sub-step (23), Constructing a fusion map: obtaining a matrix $A_{m \times n} = F_m \times F_n$ by multiplying the sample NIR spectral vector $F_m$ and the thermal analysis spectrum vector $F_n$, pooling a maximum by row vector $V_m = MAX_n(A_{m \times n}) = [v_1 \ v_2 \ ... \ v_i]$, and conducting logarithmic normalization on $V_m$ to get a fusion of a sample mapping matrix

$$S_m = \left[ \frac{e^{v_1}}{\sum_{i=1}^n e^{v_1}} \ \frac{e^{v_2}}{\sum_{i=1}^n e^{v_2}} \ \cdots \ \frac{e^{v_i}}{\sum_{i=1}^n e^{v_i}} \right]$$ , $i$ = 1, 2,..., m; and obtaining a fusion map matrix $Y$ of cigarette sample to be analyzed and $n$ single-grade tobacco leaf fusion map matrices $X = [X_1 X_2 ... X_n]$, where n is the number of single-grade tobacco samples.

Preferably, in Step (3), the sub-steps to analyze the fusion map and obtain tobacco composition and proportion of cigarettes to be analyzed are:

Sub-step (31), coding formula proportion: coding the real number for the formula proportion of each single grade tobacco leaf R = [r$_1$ r$_2$ ... r$_n$], wherein n is the number of single-grade tobacco leaf samples;

Sub-step (32), randomly initializing the coding matrix R: initializing an r value R$_1$, R$_2$, ......, R$_{200}$ to a real value between 0 and 1, wherein a sum of the values of each coding matrix should be 1, establishing a search space according to a range of more than 10 times the number of tobacco leaves composed of the formula, and randomly initializing the coding matrix, that is: R$_1$, R$_2$,...... Since the number of tobacco leaves composed of the formula is generally 10-20, the search space is established according to the 10-fold range, and 200 coding matrices are randomly initialized, namely R$_1$, R$_2$, ......R$_{200}$;

Sub-step (33), calculating a cigarette fusion spectrum matrix Z after calculating a combination of single-grade tobacco leaves in accordance with a formulation ratio R;

Sub-step (34), calculating a difference value $e$ between Z and Y by a difference correlation model of fusion maps;

Sub-step (35), Converting the difference value $e$ to a probability value P($e$);

Sub-step (36), according to the probability value, selecting a number of formula proportions to participate in a next iteration, randomly selecting two schemes for linear reorganization: $r^{(1)} = r_1 + a * (r_1 - r_2)$, and obtaining reorganized real number coding matrices R$_1$$^{(1)}$, R$_2$$^{(1)}$, ......, wherein $a$ is a scale factor generated by random numbers that obey a [-d, 1+d] uniform distribution, and d is a value that limits the scope of the reorganization;

Sub-step (37), repeating sub-steps (32) - (34) for iterative searching, and iteratively calculating e$^{(2)}$, e$^{(3)}$, e$^{(4)}$, e$^{(5)}$...... until $e$ is less than a certain value; and

Sub-step (38), ordering the probability value P(e) from largest to smallest, taking a number of formula proportions, and obtaining the tobacco composition and proportion of the cigarette to be analyzed.

Preferably, sub-step (32) should ensure that the sum of the values of each encoding matrix should be 1, and the initialization formula is as follows: $r_i = r_i / \sum_{i=1}^{n} r_i$ .

Preferably, in sub-step (33), to calculate the cigarette fusion map matrix Z after combining single-grade tobacco leaves according to the formula ratio R, the calculation formula is as follows: $Z_i = X' \times R_i$, where $R_i$ is the i-th random coding matrix, X is the tobacco fusion map matrix, and $Z_i$ is the formula fusion map matrix composed of formula proportion $R_i$.

Preferably, in sub-step (34), the formula for calculating the difference value e is as follows: $e = \sqrt{(Z-Y)' \, \Sigma^{-1} \, (Z-Y)}$ , where Y is the fusion map matrix of the cigarette sample to be analyzed, Z is the formula fusion map matrix composed of tobacco leaves in proportion, and $\Sigma$ is a covariance matrix between Y and Z.

Preferably, in sub-step (35), the calculation formula to convert the difference value e to the probability value P(e) between 0 and 1 is: $P(e) = \frac{e_{max}-e}{e_{max}-e_{min}} / sum(\frac{e_{max}-e}{e_{max}-e_{min}})$ .

Preferably, in sub-step (36), d has a value of 0.2-0.3.

Preferably, in sub-step (37), e is iteratively calculated until it is < 0.0001.

The invention has the following beneficial effects:

1. The method of the present invention designs a fusion mapping difference correlation model and a formula analysis combination optimization algorithm, automatically searches for the tobacco leaf ratio in the cigarette leaf group formula, can complete the composition analysis of any finished cigarette on the market to be analyzed within a few minutes, and can obtain a clear formula composition and proportion value, which is objective, efficient, versatile, and with good repeatability and high sensitivity. It has a unique advantage in the analysis of finished cigarettes in the tobacco industry.

2. The method of the present invention avoids wet chemical analysis of a large amount of tobacco, such as in conventional flue gas chemical composition analysis of tobacco leaf groups, and turns to dry chemical operation, which has the advantages of simple operation, minimal sample usage, within 10 mg, is non-toxic and harmless, causes no harm to the operator, and no environmental pollution.

3. The method of the present invention not only provides a fusion spectrum of the quality of the finished cigarettes to be analyzed and the single-grade tobacco leaves by near infrared and thermal analysis, but also greatly reduces the workload and the number of tests, provides concrete formula design objectives, rich data support and digital technical means for the development of cigarette products, and realizes the automatic search and objective evaluation of formula design schemes. It can avoid subjective factors and different representations that occur in traditional reliance on expert experience and sensory evaluation.

<u>EXAMPLES</u>

The present invention is further explained by embodiments below, but is not limited by the present embodiments. Experimental methods not specified in the embodiments are generally available commercially in accordance with conventional conditions, conditions described in the manual, or general equipment, materials, reagents, etc. used in accordance with conditions suggested by the manufacturer, unless otherwise specified. The following embodiments and the raw materials in the applicable ratios are commercially available.

Example: Analysis method of composition and proportion of coiled tobacco leaf group of a well-known domestic brand cigarette product sample (to be analyzed), the steps are as follows:

(1) A product sample of a well-known domestic brand of cigarettes (cigarette to be analyzed) and 50 single-grade tobacco samples of different origin, different parts and different grades of 5 grams (single-grade tobacco leaves) were selected. The cigarettes to be analyzed and single-grade tobacco leaves were screened with a 100 mesh screen and treated for 48 hours in a constant temperature and humidity environment of $(22 \pm 1)$ °C and $(60 \pm 2)$ % relative humidity.

(2) Weigh 3g sample powder and place it in a sample cup, scan the spectrum in the 4000-9000 cm$^{-1}$ band, and repeat the operation 10 times for each sample to obtain the spectral average.

(3) Before the sample thermogravimetric analysis, the thermogravimetric analyzer was set and kept at 900 °C for 10 min to clear the impurities in the furnace body, and the empty crucible was used as a reference. A $(5.00 \pm 0.05)$ mg sample was weighed and placed in a platinum thermogravimetric crucible, and the heating procedure was as follows: an initial temperature of 50 °C, a heating rate of 10 °C/min; a final temperature of 900 °C, and a constant temperature at 900 °C for 5 min. The protection gas and reaction gas were nitrogen, and the flow rate was 20 mL/min. Taking temperature (°C) as the X-axis and mass change (%) as the Y-axis, deriving the TG data, obtaining the first derivative of temperature, then obtaining the DTG data of differential weight loss, and forming the thermal analysis map.

Calculate the cigarette fusion map matrix Y and the single grade tobacco fusion atlas matrix $X = [X_1 \ X_2... \ X_{50}]$, where the number of variables of the fusion map matrix is m:

Table 1: Cigarette to be analyzed fusion map matrix Y

|  | $y_1$ | $y_2$ | $y_3$ | $y_4$ | ... | $y_m$ |
|---|---|---|---|---|---|---|
| Cigarette to be analyzed | 2.65E-05 | 3.85E-05 | 3.42E-05 | 2.02E-05 | ... | 5.48E-05 |

Table 2: Single grade tobacco fusion map matrix X

|  | $x_1$ | $x_2$ | $x_3$ | $x_4$ | ... | $x_m$ |
|---|---|---|---|---|---|---|
| Tobacco leaf 1 ($X_1$) | 3.86E-05 | 3.86E-05 | 4.51E-05 | 1.07E-05 | ... | 3.94E-05 |
| Tobacco leaf 2 ($X_2$) | 2.53E-05 | 2.53E-05 | 5.27E-05 | 1.73E-05 | ... | 2.23E-05 |
| ... | ... | ... | ... | ... | ... | ... |
| Tobacco leaf 50 ($X_{50}$) | 2.77E-05 | 2.77E-05 | 4.72E-05 | 1.96E-05 | ... | 1.78E-05 |

(4) The formula ratio real number coding matrix $R = [r_1 \ r_2 \ ... \ r_{50}]$ is set, wherein $r_1, r_2... \ r_{50}$ represents the proportion of 50 single-grade tobacco leaves used in the formulation, as shown in Table 3:

Table 3: Formula proportional real number coding matrix R

| Single grade tobacco | Formula ratio $r_i$ |
|---|---|
| Tobacco leaf 1 | $r_1$ |
| Tobacco leaf 2 | $r_2$ |
| ... | ... |
| Tobacco leaf 50 | $r_{50}$ |

(5) Initialize $r_i$ in the above table randomly to a real value between 0 and 1, and normalize $r_i$ to ensure that the sum of proportion values of each tobacco leaf is 1. The formula is as follows: $r_i = r_i / \sum_{i=1}^{n} r_i$ , where n is 50.

Table 4: Random initialization results of formula proportional real number coding matrix R

| Single grade tobacco | Formula ratio $r_i$ |
|---|---|
| Tobacco leaf 1 | 0.05 |

| Single grade tobacco | Formula ratio $r_i$ |
|---|---|
| Tobacco leaf 2 | 0.02 |
| ... | ... |
| Tobacco leaf 50 | 0.26 |
| Total | 1.00 |

(6) According to the above method, initialize at the same time 200 real number coding matrices $R_1, R_2, ......R_{200}$ to establish the formula to constitute an analytic search space, as shown in the following table:

Table 5: Formula proportional real number coding initialization

| Coding | $R_1$ | $R_2$ | $R_3$ | $R_4$ | ... | $R_{200}$ |
|---|---|---|---|---|---|---|
| $r_1$ | 0.05 | 0.14 | 0.02 | 0.00 | ... | 0.12 |
| $r_2$ | 0.02 | 0.02 | 0.01 | 0.16 | ... | 0.14 |
| $r_3$ | 0.04 | 0.03 | 0.02 | 0.22 | ... | 0.02 |
| $r_4$ | 0.03 | 0.05 | 0.08 | 0.06 | ... | 0.03 |
| ... | ... | ... | ... | ... | ... | ... |
| $r_{50}$ | 0.26 | 0.04 | 0.08 | 0.08 | ... | 0.09 |
| Total | 1.00 | 1.00 | 1.00 | 1.00 | 1.00 | 1.00 |

(7) A single grade tobacco fusion map matrix X is combined according to the formula proportion real number coding matrix R to calculate the cigarette fusion map matrix Z; $Z_i = X' \times R_i$;

Table 6: Cigarette fusion map matrix Z after combination

| | $z_1$ | $z_2$ | $z_3$ | $z_4$ | | $z_m$ |
|---|---|---|---|---|---|---|
| $Z_1$ | 4.58E-05 | 5.14E-05 | 1.17E-05 | 2.56E-05 | ... | 4.71E-05 |
| $Z_2$ | 1.27E-05 | 8.43E-06 | -2.64E-05 | 8.65E-06 | ... | 1.12E-05 |
| ... | ... | ... | ... | ... | ... | ... |
| $Z_{200}$ | 5.25E-05 | 4.78E-05 | 2.15E-05 | 2.05E-05 | ... | 2.86E-05 |

(8) A difference correlation model of the fusion map, $e = \sqrt{(Z-Y)'\, \Sigma^{-1}\, (Z-Y)}$ , is used to calculate the difference value e between Z and Y, so as to evaluate the conformity of the composition analysis of the tobacco leaf groups, as shown in Table 7 below:

Table 7: Analytical coincidence (difference between Z and Y) of coiled tobacco composition

| Candidate | $R_1$ | $R_2$ | $R_3$ | $R_4$ | ... | $R_{200}$ |
|---|---|---|---|---|---|---|
| Difference e | 4.4703 | 3.5132 | 7.18698 | 2.0721 | ... | 1.9468 |

(9) Convert the difference value e to a probability value P(e): $P(e) = \left. \dfrac{e_{max}-e}{e_{max}-e_{min}} \middle/ sum(\dfrac{e_{max}-e}{e_{max}-e_{min}}) \right.$ , as shown in Table 8 below:

Table 8: Convert the difference value e to a probability value

| Candidate | $R_1$ | $R_2$ | $R_3$ | $R_4$ | ... | $R_{200}$ | Total |
|---|---|---|---|---|---|---|---|
| Probability value P(e) | 0.51% | 0.76% | 0.59% | 1.21% | ... | 1.12% | 100% |

(10) The first 100 candidates for the formula ratio were selected randomly according to the probability values, and the formula ratios of the candidates were reorganized linearly in pairs, $r^{(1)} = r_1 + a * (r_1 - r_2)$, where a is a scale factor, generated by random numbers with a uniform distribution following [-d, 1+d], and d is 0.25, a limit so that the reorganization range is not too large.

The reorganized real number coding matrix $R_1^{(1)}, R_2^{(1)}, ...... R_{200}^{(1)}$ is obtained as shown in Table 9 below:

Table 9: Real number code of formula ratio after the first reorganization

| Code | $R_1^{(1)}$ | $R_2^{(1)}$ | $R_3^{(1)}$ | $R_4^{(1)}$ | ... | $R_{200}^{(1)}$ |
|------|------|------|------|------|------|------|
| $r_1$ | 0.00 | 0.04 | 0.20 | 0.06 | ... | 0.15 |
| $r_2$ | 0.15 | 0.12 | 0.05 | 0.15 | ... | 0.06 |
| $r_3$ | 0.05 | 0.15 | 0.06 | 0.12 | ... | 0.08 |
| $r_4$ | 0.09 | 0.25 | 0.12 | 0.05 | ... | 0.02 |
| ... | ... | ... | ... | ... | ... | ... |
| $r_{50}$ | 0.03 | 0.02 | 0.20 | 0.04 | ... | 0.08 |
| Total | 1.00 | 1.00 | 1.00 | 1.00 | 1.00 | 1.00 |

(11) According to the real number coding of the reorganized formula proportion, the reorganized cigarette fusion map matrix Z(1) was calculated, the fusion map difference association model was invoked to calculate the difference value $e^{(1)}$ between Z and Y, and the iterative calculation of $e^{(2)}$, $e^{(3)}$, $e^{(4)}$, $e^{(5)}$,......, until e = 0.000095<0.0001.

(12) According to the probability value P(e) from large to small, the top 5 formula proportional candidates are output, as shown in Table 10 below:

Table 10: Formula analysis results and P(e) values (the top 5 candidates with the highest probability values are selected)

| Code | $R_{172}^{(1)}$ | $R_{26}^{(1)}$ | $R_{23}^{(1)}$ | $R_{31}^{(1)}$ | $R_{156}^{(1)}$ |
|------|------|------|------|------|------|
| $r_1$ | 0.00 | 0.00 | 0.00 | 0.00 | 0.00 |
| $r_2$ | 0.00 | 0.02 | 0.00 | 0.08 | 0.05 |
| $r_3$ | 0.20 | 0.25 | 0.20 | 0.12 | 0.06 |
| $r_4$ | 0.15 | 0.15 | 0.05 | 0.10 | 0.25 |
| ... | ... | ... | ... | ... | ... |
| $r_{50}$ | 0.05 | 0.08 | 0.15 | 0.12 | 0.08 |
| **P(e)** | **72.05%** | **13.25%** | **4.45%** | **2.20%** | **1.15%** |

(13) According to the first five formula ratio candidates in the above table, tobacco leaves with a formula ratio of 0 are filtered out to obtain the complete composition and ratio of the tobacco leaf formula, as shown in Tables 11-15 below:

Table 11: Leaf formula corresponding to R172

| Single grade tobacco | Formula ratio |
|------|------|
| Tobacco Leaf 3 | 0.20 |
| Tobacco Leaf 4 | 0.15 |
| Tobacco Leaf 11 | 0.05 |
| Tobacco Leaf 18 | 0.05 |
| Tobacco Leaf 30 | 0.06 |
| Tobacco Leaf 31 | 0.10 |
| Tobacco Leaf 35 | 0.10 |
| Tobacco Leaf 37 | 0.05 |
| Tobacco Leaf 40 | 0.05 |
| Tobacco Leaf 42 | 0.05 |
| Tobacco Leaf 45 | 0.05 |
| Tobacco Leaf 47 | 0.02 |

(continued)

| Single grade tobacco | Formula ratio |
|---|---|
| Tobacco Leaf 49 | 0.02 |
| Tobacco Leaf 50 | 0.05 |

Table 12: Leaf formulations corresponding to R26

| Single grade tobacco | Formula ratio |
|---|---|
| Tobacco Leaf 2 | 0.02 |
| Tobacco Leaf 3 | 0.25 |
| Tobacco Leaf 4 | 0.15 |
| Tobacco Leaf 18 | 0.05 |
| Tobacco Leaf 30 | 0.02 |
| Tobacco Leaf 31 | 0.05 |
| Tobacco Leaf 35 | 0.02 |
| Tobacco Leaf 37 | 0.02 |
| Tobacco Leaf 40 | 0.05 |
| Tobacco Leaf 42 | 0.06 |
| Tobacco Leaf 45 | 0.10 |
| Tobacco Leaf 47 | 0.08 |
| Tobacco Leaf 49 | 0.05 |
| Tobacco Leaf 50 | 0.08 |

Table 13: Leaf formulations corresponding to R23

| Single grade tobacco | Formula ratio |
|---|---|
| Tobacco Leaf 3 | 0.2 |
| Tobacco Leaf 4 | 0.05 |
| Tobacco Leaf 18 | 0.08 |
| Tobacco Leaf 30 | 0.15 |
| Tobacco Leaf 31 | 0.05 |
| Tobacco Leaf 35 | 0.05 |
| Tobacco Leaf 37 | 0.02 |
| Tobacco Leaf 40 | 0.04 |
| Tobacco Leaf 42 | 0.07 |
| Tobacco Leaf 45 | 0.06 |
| Tobacco Leaf 47 | 0.10 |
| Tobacco Leaf 49 | 0.05 |
| Tobacco Leaf 50 | 0.08 |

Table 14: Leaf formulations corresponding to R31

| Single grade tobacco | Formula ratio |
|---|---|
| Tobacco Leaf 2 | 0.08 |
| Tobacco Leaf 3 | 0.12 |

(continued)

| Single grade tobacco | Formula ratio |
|---|---|
| Tobacco Leaf 4 | 0.10 |
| Tobacco Leaf 8 | 0.04 |
| Tobacco Leaf 13 | 0.10 |
| Tobacco Leaf 18 | 0.02 |
| Tobacco Leaf 19 | 0.18 |
| Tobacco Leaf 25 | 0.01 |
| Tobacco Leaf 28 | 0.08 |
| Tobacco Leaf 29 | 0.08 |
| Tobacco Leaf 40 | 0.03 |
| Tobacco Leaf 42 | 0.04 |
| Tobacco Leaf 50 | 0.12 |

Table 15: Leaf formulations corresponding to R156

| Single grade tobacco | Formula ratio |
|---|---|
| Tobacco Leaf 2 | 0.05 |
| Tobacco Leaf 3 | 0.06 |
| Tobacco Leaf 4 | 0.25 |
| Tobacco Leaf 5 | 0.05 |
| Tobacco Leaf 12 | 0.05 |
| Tobacco Leaf 14 | 0.05 |
| Tobacco Leaf 15 | 0.02 |
| Tobacco Leaf 19 | 0.02 |
| Tobacco Leaf 20 | 0.06 |
| Tobacco Leaf 21 | 0.04 |
| Tobacco Leaf 22 | 0.04 |
| Tobacco Leaf 25 | 0.08 |
| Tobacco Leaf 28 | 0.03 |
| Tobacco Leaf 29 | 0.05 |
| Tobacco Leaf 31 | 0.02 |
| Tobacco Leaf 40 | 0.02 |
| Tobacco Leaf 42 | 0.04 |
| Tobacco Leaf 50 | 0.07 |

Verification experiment: According to the five formulations shown in Tables 11-15, the single-grade tobacco leaf samples involved were mixed into cigarettes, and 9 sensory evaluation experts evaluated and scored the sensory quality differences between the mixed tobacco sample and the cigarette sample to be analyzed. The average value was taken as the actual smoking evaluation value of the quality difference, and the quality difference was qualitative after rounding. The scoring gradient settings are shown in Table 16 below:

Table 16: Sensory quality difference score gradient setting

| Quality deviation | None | slight | lesser | Intermediate | big | Large |
|---|---|---|---|---|---|---|
| Score | 0 | 1 | 2 | 3 | 4 | 5 |

The smoking evaluation results are shown in Table 17 below:

Table 17: Verification results of sensory evaluation

| Candidate formulation | P(e) | Average | Quality difference |
|---|---|---|---|
| $R_{172}$ | 72.05% | 0.00 | None |
| $R_{26}$ | 13.25% | 0.33 | Slight |
| $R_{23}$ | 4.45% | 0.44 | Slight |
| $R_{31}$ | 2.20% | 0.89 | Slight |
| $R_{156}$ | 1.15% | 1.44 | Slight |

As can be seen from Table 17, the consistency between formula ratio candidate $R_{172}$ and cigarettes to be analyzed was 72.05%, and there was no difference in sensory evaluation results. The coincidence between candidates $R_{26}, R_{23}, R_{31}, R_{156}$ and the cigarettes to be analyzed were 13.25%, 4.45%, 2.20% and 1.15%, respectively, and the results of sensory evaluation were slightly different.

The above embodiments disclose only several embodiments of the invention, and their descriptions are more specific and detailed, but they cannot be construed as limitations on the scope of the invention. Therefore, the scope of protection of the invention patent shall be subject to the attached claims.

**Claims**

1. A method for analyzing a composition and proportion of tobacco leaves in a cigarette sample based on fusion mapping, **characterized in that** it includes the following steps: (1) preparation of a cigarette sample to be analyzed and a single-grade tobacco leaf sample; (2) construction of a fusion map for the cigarette sample to be analyzed and the single-grade tobacco leaf sample; (3) analysis of the fusion map to obtain the composition and proportion of the tobacco leaves in the cigarette sample to be analyzed;
step (3), the sub-steps of the analysis of the fusion map to obtain the tobacco composition and proportion of the tobacco leaves in the cigarette to be analyzed are:

> Sub-step (31), coding formula proportion: coding a real number for a formula proportion of each single grade tobacco leaf $R = [r_1 \ r_2 \ ... \ r_n]$, where n is the number of single-grade tobacco leaf samples;
> Sub-step (32), randomly initializing a coding matrix R; initializing a value of r to a real value between 0 and 1, where a sum of the values of each coding matrix should be 1; establishing a search space according to a range of more than 10 times a number of tobacco leaves composed of the formula, and randomly initializing the coding matrix, that is: $R_1, R_2, ......$;
> Sub-step (33), calculating a cigarette fusion spectrum matrix Z after a combination of single-grade tobacco leaves in accordance with a formulation ratio R;
> Sub-step (34), calculating a difference value e between Z and Y by a difference correlation model of fusion maps;
> Sub-step (35), converting the difference value $e$ to a probability value P(e);
> Sub-step (36), according to the probability value, selecting a number of formula proportions to participate in a next iteration, randomly selecting two schemes for linear reorganization: $r^{(1)} = r_1 + a * (r_1 - r_2)$, and obtaining reorganized real number coding matrices $R_1^{(1)}, R_2^{(1)}, ......$, wherein $a$ is a scale factor generated by random numbers that obey a [-d, 1+d] uniform distribution, and d is a value that limits a scope of reorganization;
> Sub-step (37), repeating sub-steps (32) - (34) for iterative searching, and iteratively calculating $e^{(2)}, e^{(3)}, e^{(4)}, e^{(5)}......$ until e is less than a certain value; and
> Sub-step (38), ordering the probability value P(e) from largest to smallest, taking a number of formula proportions, and obtaining the tobacco composition and proportion of the cigarette to be analyzed.

2. The method of Claim 1, **characterized in that** step (1) includes a single cigarette sample to be analyzed and at least 50 single-grade tobacco leaf samples; each sample is placed in a constant temperature and humidity environment of (22 $\pm$ 1) °C and (60 $\pm$ 2) % relative humidity for at least 48 hours.

3. The method of claim 1, **characterized in that** step (2) the sub-steps of the fusion map construction of the cigarette sample to be analyzed and single-grade tobacco sample are as follows:

Sub-step (21), collecting a near-infrared spectrum: weigh 3 g sample powder and place it in a sample cup, scan a spectrum in a 4000-9000 cm$^{-1}$ band, and repeat 10 times for each sample to obtain a spectral average;

Sub-step (22), collecting a thermal analysis map: samples are respectively placed in thermogravimetric (TG) crucibles and heated according to a procedure including an initial temperature of 50 °C, a heating rate of 10 °C/min, a final temperature of 900 °C, and a constant temperature at 900 °C for 5min; the protection gas and reaction gas are nitrogen, and a flow rate is 20 mL/min; taking temperature (°C) as an X-axis and mass change (%) as a Y-axis, deriving TG data, obtaining a first derivative of temperature, then obtaining derivative thermogravimetric (DTG) data of differential weight loss, and forming the thermal analysis map; and

Sub-step (23), constructing the fusion map: obtaining a matrix $A_{m \times n} = F_m \times F_n$ by multiplying a near infrared (NIR) spectral vector $F_m$ of the sample and the thermal analysis spectrum vector $F_n$, pooling a maximum by row vector $V_m = MAX_n(A_{m \times n}) = [v_1\ v_2\ ...\ v_i]$, and logarithmically normalizing $V_m$ to get a fusion of a sample mapping matrix

$$s_m = \left[ \frac{e^{v_1}}{\sum_{i=1}^{n} e^{v_1}}\ \frac{e^{v_2}}{\sum_{i=1}^{n} e^{v_2}}\ \cdots\ \frac{e^{v_i}}{\sum_{i=1}^{n} e^{v_i}} \right], = 1, 2, \ldots, \mathrm{m}$$ ; obtaining a fusion map matrix $Y$ of the cigarette sample to be analyzed, and $n$ single-grade tobacco leaf fusion map matrices $X = [X_1\ X_2\ ...\ X_n]$, where $n$ is a number of the single-grade tobacco samples.

4. The method of claim 1, **characterized in that** sub-step (32) should ensure that the sum of the values of each encoding matrix should be 1, and the initialization formula is as follows: $r_i = r_i / \sum_{i=1}^{n} r_i$ .

5. The method of Claim 1, **characterized in that** in sub-step (33), to calculate the cigarette fusion map matrix Z after combining single-grade tobacco leaves according to the formula ratio R, the calculation formula is as follows: $Z_i = X' \times R_i$, wherein $R_i$ is the i-th random coding matrix, X is the tobacco fusion map matrix, and $Z_i$ is the formula fusion map matrix composed of formula proportion $R_i$.

6. The method of Claim 1, **characterized in that** in sub-step (34), the formula for calculating the difference value e is as follows: $e = \sqrt{(Z - Y)' \Sigma^{-1} (Z - Y)}$ , wherein Y is the fusion map matrix of the cigarette to be analyzed, Z is the formula fusion map matrix composed of tobacco leaves in proportion, and $\Sigma$ is a covariance matrix between Y and Z.

7. The method of Claim 1, **characterized in that** in sub-step (35), a calculation formula to convert the difference value e value to the probability value P(e) between 0 and 1 is: $P(e) = \frac{e_{max} - e}{e_{max} - e_{min}} / sum\left( \frac{e_{max} - e}{e_{max} - e_{min}} \right)$ .

8. The method of Claim 1, **characterized in that** in sub-step (36), d has a value of 0.2-0.3.

9. The method of Claim 1, **characterized in that** in sub-step (37), $e$ is iteratively calculated until it is < 0.0001.

## Patentansprüche

1. Ein Verfahren zur Analyse einer Zusammensetzung und eines Anteils von Tabakblättern in einer Zigarettenprobe auf der Grundlage von Fusionskartierung, **dadurch gekennzeichnet, dass** es die folgenden Schritte enthält: (1) Vorbereitung einer zu analysierenden Zigarettenprobe und einer sortenreinen Tabakblattprobe; (2) Erstellung einer Fusionskarte für die zu analysierende Zigarettenprobe und die sortenreine Tabakblattprobe; (3) Analyse der Fusionskarte, um die Zusammensetzung und den Anteil der Tabakblätter in der zu analysierenden Zigarettenprobe zu erhalten;

Schritt (3): Die Unterschritte der Analyse der Fusionskarte zur Ermittlung der Tabakzusammensetzung und des Anteils der Tabakblätter in der zu analysierenden Zigarette sind:

Unterschritt (31), Codierung eines Formelanteils: Kodierung einer reellen Zahl für einen Formelanteil jedes sortenreinen Tabakblatts R = [$r_1$ $r_2$ ... $r_n$], wobei n die Anzahl der sortenreinen Tabakblattproben ist;

Unterschritt (32), zufällige Initialisierung einer Codierungsmatrix R; Initialisierung eines Wertes von r auf einen reellen Wert zwischen 0 und 1, wobei eine Summe der Werte jeder Codierungsmatrix 1 sein sollte; Einrichtung eines Suchraums gemäß einem Bereich von mehr als dem Zehnfachen einer Anzahl von Tabakblättern, die aus der Formel bestehen, und zufällige Initialisierung der Codierungsmatrix, d. h.: $R_1$, $R_2$,......;

Unterschritt (33), Berechnen einer Zigaretten-Fusionsspektrummatrix Z nach einer Kombination sortenreiner Tabakblätter gemäß einem Formulierungsverhältnis R;

Unterschritt (34), Berechnen eines Differenzwerts e zwischen Z und Y durch ein Differenzkorrelationsmodell von Fusionskarten;

Unterschritt (35), Umwandeln des Differenzwerts e in einen Wahrscheinlichkeitswert P(e);

Unterschritt (36), Auswählen einer Anzahl von Formelanteilen gemäß dem Wahrscheinlichkeitswert, die an einer nächsten Iteration teilnehmen sollen, zufälliges Auswählen von zwei Schemata für die lineare Reorganisation: $r^{(1)} = r_1 + a * (r_1 - r_2)$, und Erhalten von reorganisierten reellen Zahlen-Codierungsmatrizen $R_1^{(1)}$, $R_2^{(1)}$, ......, wobei a ein Skalierungsfaktor ist, der durch Zufallszahlen erzeugt wird, die einer gleichmäßigen Verteilung [-d, 1+d] gehorchen, und d ein Wert ist, der den Umfang der Reorganisation begrenzt;

Unterschritt (37), Wiederholen von Unterschritten (32) - (34) für die iterative Suche und iteratives Berechnen von $e^{(2)}$, $e^{(3)}$, $e^{(4)}$, $e^{(5)}$...... bis e kleiner als ein bestimmter Wert ist; und

Unterschritt (38), Ordnen des Wahrscheinlichkeitswerts P(e) vom größten zum kleinsten, Nehmen einer Anzahl von Formelanteilen und Ermitteln der Tabakzusammensetzung und des Anteils der zu analysierenden Zigarette.

2. Das Verfahren nach Anspruch 1, **dadurch gekennzeichnet, dass** Schritt (1) eine einzelne zu analysierende Zigarettenprobe und mindestens 50 sortenreine Tabakblattproben enthält; jede Probe wird mindestens 48 Stunden lang in einer Umgebung mit konstanter Temperatur und Luftfeuchtigkeit von (22 $\pm$ 1) °C und (60 $\pm$ 2) % relativer Luftfeuchtigkeit aufbewahrt.

3. Das Verfahren nach Anspruch 1, **dadurch gekennzeichnet, dass** in Schritt (2) die Unterschritte der Fusionskartenerstellung der zu analysierenden Zigarettenprobe und der sortenreinen Tabakprobe wie folgt sind:

Unterschritt (21), Erfassen eines Nahinfrarotspektrums: 3 g Probenpulver wiegen und in einen Probenbecher geben, ein Spektrum in einem Band von 4000-9000 cm$^{-1}$ scannen und für jede Probe 10-mal wiederholen, um einen Spektralmittelwert zu erhalten;

Unterschritt (22), Erfassen einer thermischen Analysekarte: Die Proben werden jeweils in thermogravimetrische (TG) Tiegel gegeben und gemäß einem Verfahren erhitzt, das eine Anfangstemperatur von 50 °C, eine Heizrate von 10 °C/min, eine Endtemperatur von 900 °C und eine konstante Temperatur von 900 °C für 5 min enthält; das Schutzgas und das Reaktionsgas sind Stickstoff, und die Durchflussrate beträgt 20 mL/min; Verwenden der Temperatur (°C) als X-Achse und der Massenänderung (%) als Y-Achse, Ableiten von TG-Daten, Erhalten einer ersten Ableitung der Temperatur, dann Erhalten abgeleiteter thermogravimetrischer (DTG) Daten des differentiellen Gewichtsverlusts und Bilden der thermischen Analysekarte; und

Unterschritt (23), Bilden der Fusionskarte: Erhalten einer Matrix $A_{m \times n} = F_m \times F_n$ durch Multiplikation eines Nahinfrarot (NIR)-Spektralvektors $F_m$ der Probe und des thermischen Analysespektrumvektors $F_n$, Zusammen-fassen eines Maximums durch den Zeilenvektor $V_m = MAX_n(A_{m \times n}) = [v_1\ v_2\ ...\ v_i]$, und logarithmische Norma-lisierung von $V_m$, um eine Fusion einer Probenkartierungsmatrix

$$s_m = \left[\frac{e^{v_1}}{\sum_{i=1}^{n} e^{v_1}} \ \frac{e^{v_2}}{\sum_{i=1}^{n} e^{v_2}} \ \cdots \ \frac{e^{v_i}}{\sum_{i=1}^{n} e^{v_i}}\right], = 1, 2, \ldots, \ \mathrm{m}$$ ; zu bekommen; Erhalten einer Fusionskartenmatrix Y der zu analysierenden Zigarettenprobe und n sortenreiner Tabakblatt-Fusionskartenmatrizen $X = [X_1\ X_2\ ...\ X_n]$, wobei n eine Anzahl der sortenreinen Tabakproben ist.

4. Das Verfahren nach Anspruch 1, **dadurch gekennzeichnet, dass** Unterschritt (32) sicherstellen sollte, dass die Summe der Werte jeder Codierungsmatrix 1 sein sollte, und die Initialisierungsformel wie folgt lautet: $r_i = r_i / \sum_{i=1}^{n} r_i$ .

5. Das Verfahren nach Anspruch 1, **dadurch gekennzeichnet, dass** in Unterschritt (33) zur Berechnung der Zigaretten-Fusionskartenmatrix Z nach dem Mischen sortenreiner Tabakblätter gemäß dem Formelverhältnis R die folgende Berechnungsformel verwendet wird: $Z_i = X' \times R_i$, wobei $R_i$ die i-te Zufallscodierungsmatrix, X die Tabak-Fusions-kartenmatrix und $Z_i$ die Formel-Fusionskartenmatrix ist, die aus dem Formelverhältnis $R_i$ zusammengesetzt ist.

**6.** Das Verfahren nach Anspruch 1, **dadurch gekennzeichnet, dass** in Unterschritt (34) die Formel zur Berechnung des Differenzwerts e wie folgt lautet: $e = \sqrt{(Z-Y)\,\Sigma^{-1}\,(Z-Y)}$ , wobei Y die Fusionskartierungsmatrix der zu analysierenden Zigarette ist, Z die Formel-Fusionskartierungsmatrix ist, die aus Tabakblättern im Verhältnis zusammengesetzt ist, und $\Sigma$ eine Kovarianzmatrix zwischen Y und Z ist.

**7.** Das Verfahren nach Anspruch 1, **dadurch gekennzeichnet, dass** in Unterschritt (35) eine Berechnungsformel zum Umrechnen des Differenzwerts *e* in den Wahrscheinlichkeitswert P(e) zwischen 0 und 1 lautet:

$$P(e) = \frac{e_{max}-e}{e_{max}-e_{min}} \bigg/ sum\left(\frac{e_{max}-e}{e_{max}-e_{min}}\right) .$$

**8.** Das Verfahren nach Anspruch 1, **dadurch gekennzeichnet, dass** in Unterschritt (36) d einen Wert von 0,2 bis 0,3 hat.

**9.** Das Verfahren nach Anspruch 1, **dadurch gekennzeichnet, dass** in Unterschritt (37) *e* iterativ berechnet wird, bis es < 0,0001 ist.

## Revendications

**1.** Méthode pour analyser une composition et une proportion de feuilles de tabac dans un échantillon de cigarette basée sur une cartographie de fusion, **caractérisée en ce qu'**elle inclut les étapes suivantes : (1) préparation d'un échantillon de cigarette à analyser et d'un échantillon de feuille de tabac de qualité unique ; (2) construction d'une carte de fusion pour l'échantillon de cigarette à analyser et l'échantillon de feuilles de tabac de qualité unique ; (3) analyse de la carte de fusion pour obtenir la composition et la proportion des feuilles de tabac dans l'échantillon de cigarette à analyser ;
étape (3), les sous-étapes de l'analyse de la carte de fusion pour obtenir la composition et la proportion des feuilles de tabac dans la cigarette à analyser sont :

Sous-étape (31), proportion de formule de codage : coder un nombre réel pour une proportion de formule de chaque feuille de tabac de qualité unique R = [$r_1$ $r_2$ ... $r_n$], où n est le nombre d'échantillons de feuilles de tabac de qualité unique ;
Sous-étape (32), initialisation aléatoire d'une matrice de codage R ; initialiser une valeur de r en une valeur réelle entre 0 et 1, où la somme des valeurs de chaque matrice de codage devrait être 1 ; établir un espace de recherche selon une plage de plus de 10 fois un nombre de feuilles de tabac composées de la formule, et initialiser aléatoirement la matrice de codage, c'est-à-dire : $R_1$, $R_2$, ...... ;
Sous-étape (33), calculer une matrice spectrale de fusion de cigarette Z après une combinaison de feuilles de tabac de qualité unique selon un ratio de formulation R;
Sous-étape (34), calculer une valeur de différence e entre Z et Y par un modèle de corrélation de différence des cartes de fusion ;
Sous-étape (35), convertir la valeur de différence e en une valeur de probabilité P(e) ;
Sous-étape (36), selon la valeur de probabilité, sélectionner un nombre de proportions de formule pour participer dans une itération suivante, sélectionner aléatoirement deux schémas de réorganisation linéaire : $r^{(1)} = r_1 + a * (r_1 - r_2)$, et obtenir des matrices de codage de nombre réel réorganisées $R_1^{(1)}$, $R_2^{(1)}$, ....., dans laquelle a est un facteur d'échelle généré par des nombres aléatoires qui obéissent à une distribution uniforme [-d, 1+d], et d est une valeur qui limite un champ de réorganisation ;
Sous-étape (37), répéter sous-étapes (32) - (34) pour la recherche itérative, et calculer itérativement $e^{(2)}$, $e^{(3)}$, $e^{(4)}$, $e^{(5)}$...... jusqu'à e est inférieur à une certaine valeur ; et
Sous-étape (38), ordonner la valeur de probabilité P(e) du plus grand au plus petit, prendre un nombre de proportions de formule, et obtenir la composition et la proportion de tabac de la cigarette à analyser.

**2.** La méthode de la Revendication 1, **caractérisée en ce que** l'étape (1) inclut un seul échantillon de cigarette à analyser et au moins 50 échantillons de feuilles de tabac de qualité unique ; chaque échantillon est placé dans un environnement à température et humidité constantes de (22 $\pm$ 1) °C et (60 $\pm$ 2) % d'humidité relative pendant au moins 48 heures.

**3.** La méthode de la revendication 1, **caractérisée en ce qu'**étape (2) les sous-étapes de la construction de carte de fusion de l'échantillon de cigarette à analyser et de l'échantillon de tabac de qualité unique sont les suivantes :

Sous-étape (21), collecter un spectre proche infrarouge : peser 3 g de poudre d'échantillon et la placer dans un gobelet d'échantillon, balayer un spectre dans une bande de 4000 - 9000 cm$^{-1}$, et répéter 10 fois pour chaque échantillon pour obtenir une moyenne spectrale ;

Sous-étape (22), collecte d'une carte d'analyse thermique : les échantillons sont respectivement placés dans des creusets thermogravimétriques (TG) et chauffés selon une procédure comprenant une température initiale de 50 °C, un taux de chauffage de 10 °C/min, une température finale de 900 °C et une température constante de 900 °C pendant 5 minutes ; le gaz de protection et le gaz de réaction sont de l'azote, et un débit est de 20 mL/min ; prendre la température (°C) comme axe X et la variation de masse (%) comme axe Y, dériver des données TG, obtenir une première dérivée de température, puis obtenir des données dérivées thermogravimétriques (DTG) de perte de poids différentielle, et former la carte d'analyse thermique ; et

Sous-étape (23), construction de la carte de fusion : obtenir une matrice $A_{m \times n} = F_m \times F_n$ en multipliant un vecteur spectral proche infrarouge (NIR) $F_m$ de l'échantillon et le vecteur spectral d'analyse thermique $F_n$, regrouper un maximum par vecteur ligne $V_m = MAX_n (A_{m \times n}) = [v_1\ v_2 ... v_i]$, et normaliser logarithmiquement $V_m$ pour obtenir une fusion d'une matrice de cartographie d'échantillon

$$S_m = \left[ \frac{e^{v_1}}{\sum_{i=1}^{n} e^{v_1}} \frac{e^{v_2}}{\sum_{i=1}^{n} e^{v_2}} \cdots \frac{e^{v_i}}{\sum_{i=1}^{n} e^{v_i}} \right], = 1, 2, ..., m$$

; obtenir une matrice de carte de fusion Y de l'échantillon de cigarette à analyser, et $n$ matrices de carte de fusion de feuille de tabac de qualité unique $X = [X_1\ X_2 ... X_n]$, où $n$ est un nombre des échantillons de tabac de qualité unique.

4. La méthode de la revendication 1, **caractérisée en ce que** sous-étape (32) devrait garantir que la somme des valeurs de chaque matrice d'encodage devrait être 1, et la formule d'initialisation est la suivante :

$$r_i = r_i / \sum_{i=1}^{n} r_i .$$

5. La méthode de la Revendication 1, **caractérisée en ce que** dans la sous-étape (33), pour calculer la matrice de carte de fusion de cigarette Z après combinaison des feuilles de tabac de qualité unique selon le ratio de formule R, la formule de calcul est la suivante : $Z_i = X' \times R_i$, dans laquelle $R_i$ est la i-ème matrice de codage aléatoire, X est la matrice de carte de fusion de tabac, et $Z_i$ est la matrice de carte de fusion de formule composée de la proportion de formule $R_i$.

6. La méthode de la Revendication 1, **caractérisée en ce que** dans la sous-étape (34), la formule pour calculer la valeur de différence e est la suivante : $e = \sqrt{(Z - Y)' \sum^{-1} (Z - Y)}$, dans laquelle Y est la matrice de carte de fusion de cigarette à analyser, Z est la matrice de carte de fusion de formule composée de feuilles de tabac en proportion, et $\Sigma$ est une matrice de covariance entre Y et Z.

7. La méthode de la Revendication 1, **caractérisée en ce que** dans la sous-étape (35), une formule de calcul pour convertir la valeur de différence e en la valeur de probabilité P(e) entre 0 et 1 est :

$$P(e) = \frac{\frac{e_{max} - e}{e_{max} - e_{min}}}{sum(\frac{e_{max} - e}{e_{max} - e_{min}})} .$$

8. La méthode de la Revendication 1, **caractérisée en ce que** dans la sous-étape (36), d a une valeur de 0,2 - 0,3.

9. La méthode de la Revendication 1, **caractérisée en ce que** dans la sous-étape (37), e est calculé itérativement jusqu'à ce qu'il est < 0,0001.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• CN 116482056